# EUROPEAN PATENT APPLICATION

(11) **EP 4 681 711 A1**
(43) Date of publication of application: **21.01.2026**
(21) Application number: 24770996.7
(22) Date of filing: 15.03.2024
(51) Int. Cl.: A61K 31/426, A61P 13/02, A61P 13/10

(54) **DYSURIA-ALLEVIATING AGENT**

(30) Priority: 15.03.2023 JP 2023040926
(71) Applicant: Zeria Pharmaceutical Co., Ltd., Tokyo 103-8351 (JP)
(72) Inventor: OKUBO, Takanori, Tokyo 103-8351 (JP); MIYAGAWA, Tomoharu, Tokyo 103-8351 (JP); NAGAHAMA, Kenji, Tokyo 103-8351 (JP)
(74) Representative: Wächtershäuser & Hartz Patentanwaltspartnerschaft mbB
(86) International application number: PCT/JP2024/010168
(87) International publication number: WO 2024/190894

(57) **Abstract**

An object of the present invention is to develop a urination disorder-alleviating agent which has a sufficient urination disorder improving effect and can be administered chronically.

The present invention relates to a urination disorder-alleviating agent comprising acotiamide or a salt thereof as an active ingredient, wherein the acotiamide or a salt thereof is orally administered in an amount of 200 to 300 mg per administration for a total of 600 to 900 mg per day to an adult.

## Description

### Technical Field

The present invention relates to a pharmaceutical agent for alleviating urination disorder caused by conditions such as underactive bladder.

### Background Art

Urination disorder is roughly classified into urine storage dysfunction and urinary voiding dysfunction. Urine storage dysfunction is a disorder of the function of storing urine, and includes frequent urination and urinary incontinence (such as urge urinary incontinence and stress urinary incontinence). Meanwhile, urinary voiding dysfunction is a disorder during urination, and examples thereof include difficulty in urination, residual urine, and urinary retention. Each pathogenesis can be classified as either neurogenic or non-neurogenic, but can be regarded as a functional abnormality such as excessive contraction or incomplete contraction of the bladder smooth muscle (detrusor smooth muscle).

Among the urine storage dysfunctions, "the syndrome characterized primarily by urinary urgency, usually accompanied by frequent urination and nocturia, and in some cases resulting in urge urinary incontinence", was defined as overactive bladder (OAB) by the International Continence Society (ICS) in 2002. Meanwhile, underactive bladder (UAB) is a condition in which "the bladder contraction during urination is incomplete, resulting in symptoms such as difficulty in urination", which is a symptom syndrome that suggests detrusor underactivity.

For the treatment of urination disorder due to underactive bladder and overactive bladder, cholinergic agents (muscarinic receptor agonists and cholinesterase inhibitors) are used since aging reduces the expression level of muscarinic M3 receptors in the bladder smooth muscle and decreases the amount of acetylcholine secretion. That is, it is known that the cholinergic agents enhance detrusor muscle contraction and exhibit an effect of improving voiding function by acting directly or indirectly on muscarinic M3 receptors of the bladder smooth muscle. When no effect is observed with cholinergic agents or when the physician determines that the use of cholinergic agents is not appropriate, management is performed by intermittent self-catheterization or indwelling catheterization.

Acotiamide has a chemical name of N-[2-[bis(1-methylethyl)amino]ethyl]-2-[(2-hydroxy-4,5-dimethoxybenzoyl)amino]thiazole-4-carboxamide, and a molecular formula of C₂₁H₃₀N₄O₅S. Acotiamide is known to improve gastrointestinal motility disorders by significantly enhancing gastrointestinal motility (Patent Literature 1), and its hydrochloride hydrate (Acofide) is commercially available as a therapeutic agent for functional dyspepsia.

In addition, as a result of conducting a pilot study in which acotiamide (100 mg, orally administered three times a day for two weeks) was used in combination with an α1-receptor blocker in patients with underactive bladder, the mean value of residual urine volume after administration did not fall below 100 mL, which is regarded as requiring specialized treatment according to the clinical guidelines for lower urinary tract symptoms (Non-Patent Literature 1 to 3). The effect of acotiamide on the underactive bladder model in rats was investigated only at doses (up to 100 mg/kg, subcutaneous administration) that exhibit gastrointestinal motility improving action, and the efficacy and tolerability of high-dose administration greatly exceeding this dose were not examined (Non-Patent Literature 4).

### Citation List

### Patent Literature

Patent Literature **1:** WO 1996/36619 A

### Non-Patent Literature

Non-Patent Literature **1:** Res. Rep. Urol. 2015; 7:81-83
Non-Patent Literature **2:** Res. Rep. Urol. 2017; 9:141-143
Non-Patent Literature **3:** Int. J. Urol. 2019; 26(8):848-849
Non-Patent Literature **4:** The Journal of Japanese Continence Society. 2022; 33(1):224

### Summary of Invention

### Technical Problem

However, with conventional cholinergic agent therapy, there are problems such as insufficient improvement in voiding function, attenuation of the voiding improvement effect with long-term administration, decreased urine storage capacity due to reduced bladder compliance, and the possibility of worsening overactive bladder symptoms in patients with urination disorder accompanied by overactive bladder. In addition, cholinergic agents are generally known to cause cholinergic adverse effects such as diarrhea, abdominal pain, and sweating, and may induce cholinergic crisis as a serious adverse effect, thus requiring caution in their use. Cholinergic crisis refers to a sudden deterioration of the acetylcholine excess state accompanied by respiratory distress that occurs during administration of cholinesterase (ChE) inhibitors, and indicates a condition requiring mechanical ventilation.

Therefore, it is desired to develop a urination disorder-alleviating agent which has a sufficient urination disorder alleviating effect and can be administered chronically.

### Solution to Problem

The present inventors, as a result of various investigations to solve the above problems, have completed the present invention upon finding, quite unexpectedly, that acotiamide or a salt thereof, which did not exhibit a sufficient effect even when used in combination with an α1-receptor blocker as described above, exhibited excellent urination disorder alleviating action when administered alone in a high oral dose without causing serious adverse effects, and being suitable for long-term administration.

That is, the present invention provides the following inventions [1] to [16].
[1] A urination disorder-alleviating agent comprising acotiamide or a salt thereof as an active ingredient, wherein the acotiamide or a salt thereof is orally administered in an amount of 200 to 300 mg per administration for a total of 600 to 900 mg per day to an adult.
[2] The urination disorder-alleviating agent according to [1], wherein an administration subject is a patient selected from the group consisting of patients with underactive bladder and patients with underactive bladder accompanied by overactive bladder.
[3] The urination disorder-alleviating agent according to [1] or [2], wherein the number of oral administrations of acotiamide or a salt thereof per day is 2 to **3.**
[4] The urination disorder-alleviating agent according to any one of [1] to [3], wherein an administration subject is a patient with underactive bladder.
[5] Use of acotiamide or a salt thereof for producing a urination disorder-alleviating agent, wherein the acotiamide or a salt thereof is orally administered in an amount of 200 to 300 mg per administration for a total of 600 to 900 mg per day to an adult.
[6] The use according to [5], wherein an administration subject is a patient selected from the group consisting of patients with underactive bladder and patients with underactive bladder accompanied by overactive bladder.
[7] The use according to [5], wherein the number of oral administrations of acotiamide or a salt thereof per day is 2 to **3.**
[8] The use according to any one of [5] to [7], wherein an administration subject is a patient with underactive bladder.
[9] Acotiamide or a salt thereof for use in alleviating urination disorder, wherein the acotiamide or a salt thereof is orally administered in an amount of 200 to 300 mg per administration for a total of 600 to 900 mg per day to an adult.
[10] The acotiamide or a salt thereof according to [9], wherein an administration subject is a patient selected from the group consisting of patients with underactive bladder and patients with underactive bladder accompanied by overactive bladder.
[11] The acotiamide or a salt thereof according to [9] or [10], wherein the number of oral administrations per day is 2 to **3.**
[12] The acotiamide or a salt thereof according to any one of [9] to [11], wherein an administration subject is a patient with underactive bladder.
[13] A method for alleviating urination disorder, comprising orally administering acotiamide or a salt thereof in an amount of 200 to 300 mg per administration for a total of 600 to 900 mg per day to an adult.
[14] The method according to [13], wherein an administration subject is a patient selected from the group consisting of patients with underactive bladder and patients with underactive bladder accompanied by overactive bladder.
[15] The method according to [13], wherein the number of oral administrations of acotiamide or a salt thereof per day is 2 to **3.**
[16] The method according to [13], wherein an administration subject is a patient with underactive bladder.

### Advantageous Effects of Invention

When acotiamide or a salt thereof is orally administered to an adult in an amount of 200 to 300 mg per administration for a total of 600 to 900 mg, the urination disorder in a patient with underactive bladder and/or a patient with overactive bladder is remarkably improved, serious adverse effects do not occur, and long-term administration is also possible.

### Brief Description of Drawings

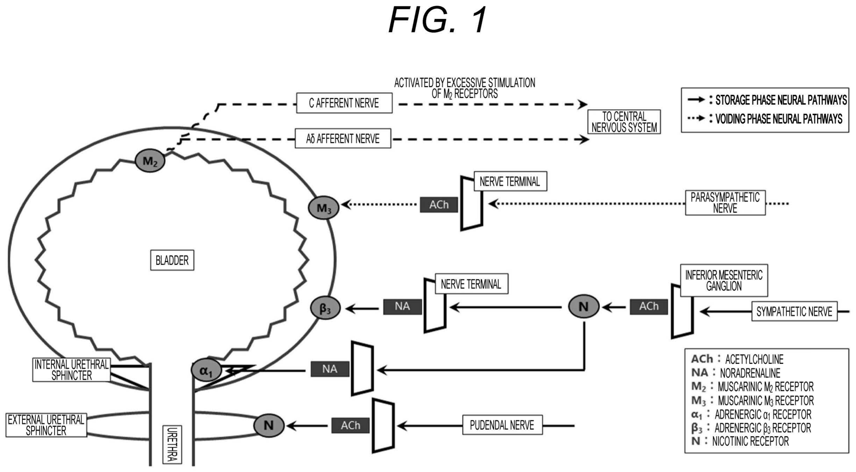

Fig. 1 is a view showing an outline of lower urinary tract innervation.

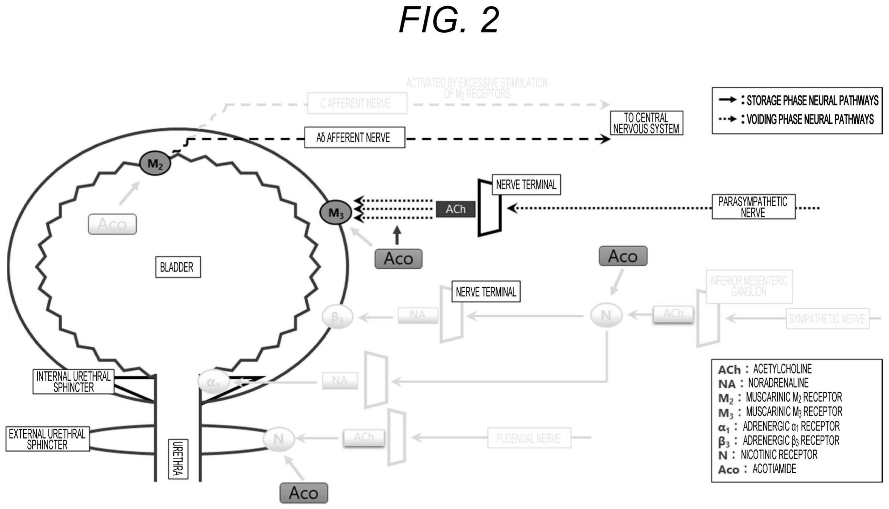

Fig. 2 shows a lower urinary tract nerve response upon administration of acotiamide.

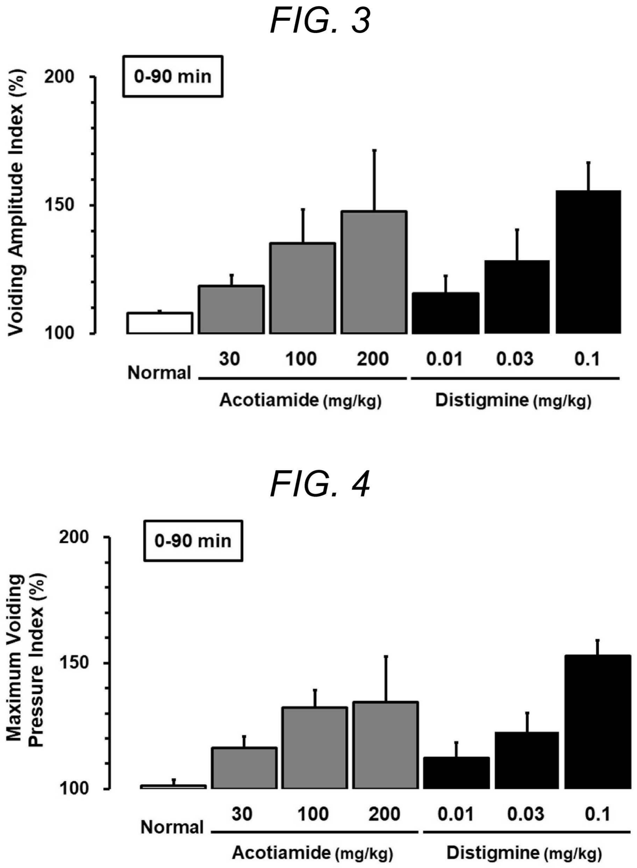

Fig. 3 shows results of bladder contraction pressure during voiding in Example 1 (0 to 90 minutes after administration). In the drawing, the bar graph shows values of a normal group (Normal), acotiamide administration groups (30 to 200 mg/kg), and distigmine administration groups (0.01 to 0.1 mg/kg), respectively, from the left. The vertical axis represents the rate of change in bladder contraction pressure during voiding (voiding amplitude index, %) (mean value and standard error of 4 to 5 animals in each group).
Fig. 4 shows results of maximum contraction pressure during voiding in Example 1 (0 to 90 minutes after administration). In the drawing, the bar graph shows values of a normal group (Normal), acotiamide administration groups (30 to 200 mg/kg), and distigmine administration groups (0.01 to 0.1 mg/kg), respectively, from the left. The vertical axis represents the rate of change in maximum contraction pressure during voiding (maximum voiding pressure index, %) (mean value and standard error of 4 to 5 animals in each group).

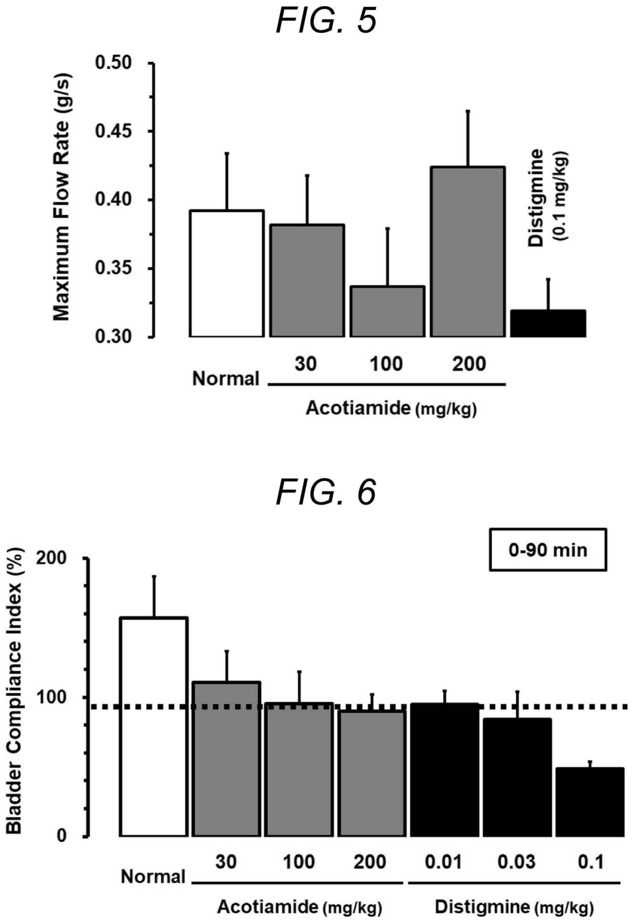

Fig. 5 shows results of urine flow rate in Example **1.** In the drawing, the bar graph shows values of a normal group (Normal), acotiamide administration groups (30 to 200 mg/kg), and a distigmine administration group (0.1 mg/kg), respectively, from the left. The vertical axis represents the maximum urine flow rate (maximum flow rate, g/s) (mean value and standard error of 6 animals in each group).
Fig. 6 shows results of bladder compliance in Example 1 (0 to 90 minutes after administration). In the drawing, the bar graph shows values of a normal group (Normal), acotiamide administration groups (30 to 200 mg/kg), and distigmine administration groups (0.01 to 0.1 mg/kg), respectively, from the left. The vertical axis represents the rate of change in bladder compliance (bladder compliance index, %) (mean value and standard error of 4 to 5 animals in each group). The dotted line shows a lower limit value of the bladder compliance change rate (mean value) of the normal animal group in this study.

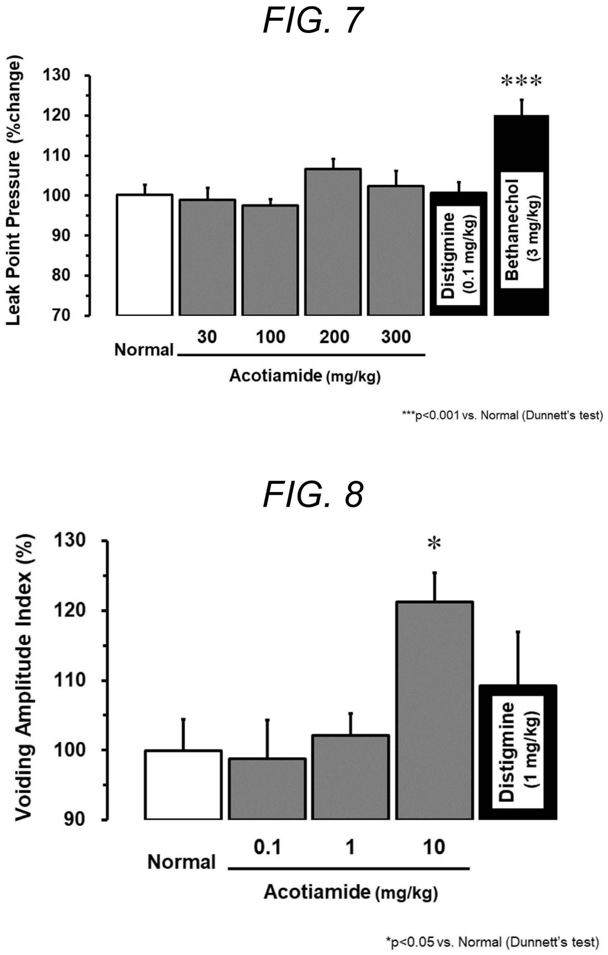

Fig. 7 shows results of leak point pressure in Example **3.** In the drawing, the bar graph shows values of a normal group (Normal), acotiamide administration groups (30 to 300 mg/kg), a distigmine administration group (0.1 mg/kg), and bethanechol administration group (3 mg/kg), respectively, from the left. The vertical axis represents the rate of change (%) in the leak point pressure (mean value and standard error of 6 to 7 animals in each group).
Fig. 8 shows results of bladder contraction pressure during voiding in Example 4 (0 to 30 minutes after administration). In the drawing, the bar graph shows values of a normal group (Normal), acotiamide administration groups (0.1 to 10 mg/kg), and a distigmine administration group (1 mg/kg), respectively, from the left. The vertical axis represents the rate of change in bladder contraction pressure during voiding (voiding amplitude index, %) (mean value and standard error of 8 animals in each group).

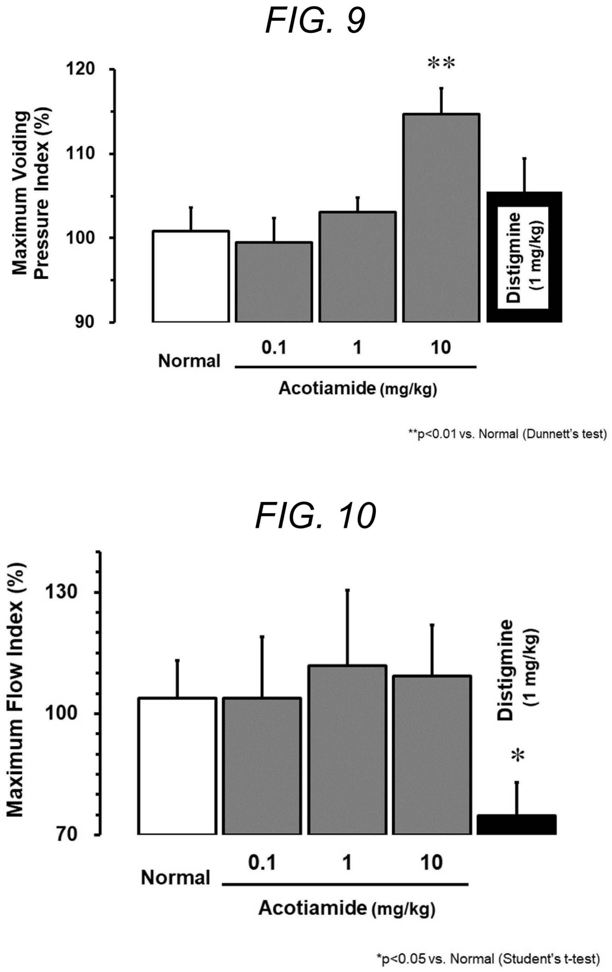

Fig. 9 shows results of maximum contraction pressure during voiding in Example 4 (0 to 30 minutes after administration). In the drawing, the bar graph shows values of a normal group (Normal), acotiamide administration groups (0.1 to 10 mg/kg), and a distigmine administration group (1 mg/kg), respectively, from the left. The vertical axis represents the rate of change in maximum contraction pressure during voiding (voiding amplitude index, %) (mean value and standard error of 8 animals in each group).
Fig. 10 shows results of urine flow rate (maximum urine flow rate) in Example 4 (0 to 30 minutes after administration). In the drawing, the bar graph shows values of a normal group (Normal), acotiamide administration groups (0.1 to 10 mg/kg), and a distigmine administration group (1 mg/kg), respectively, from the left. The vertical axis represents the rate of change in maximum urine flow rate during voiding (maximum flow index, %) (mean value and standard error of 8 animals in each group).

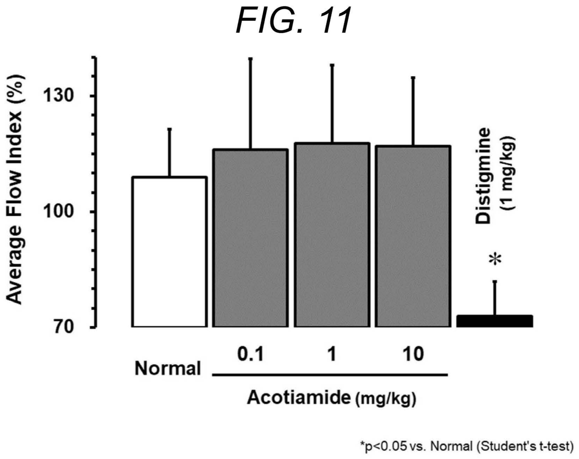

Fig. 11 shows results of urine flow rate (average urine flow rate) in Example 4 (0 to 30 minutes after administration). In the drawing, the bar graph shows values of a normal group (Normal), acotiamide administration groups (0.1 to 10 mg/kg), and a distigmine administration group (1 mg/kg), respectively, from the left. The vertical axis represents the rate of change in average urine flow rate (average flow index, %) (mean value and standard error of 8 animals in each group).

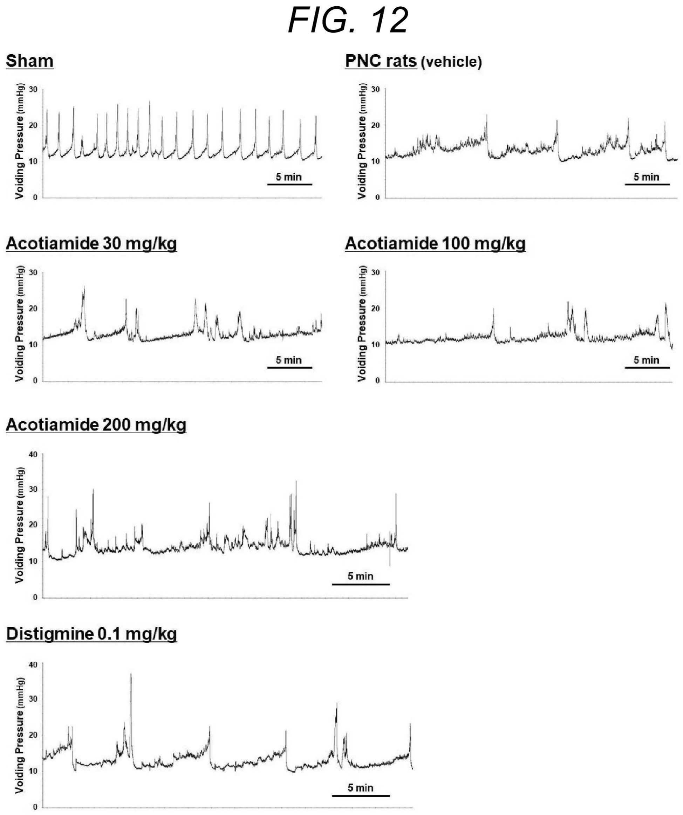

Fig. 12 shows results of detrusor muscle contraction pressure in Example **5.** Traces of detrusor muscle contraction pressure are shown for a sham-operated group, a pelvic nerve crush injury group, acotiamide administration groups (30 to 200 mg/kg), and a distigmine administration group (0.1 mg/kg), respectively (30 to 60 minutes after drug administration).

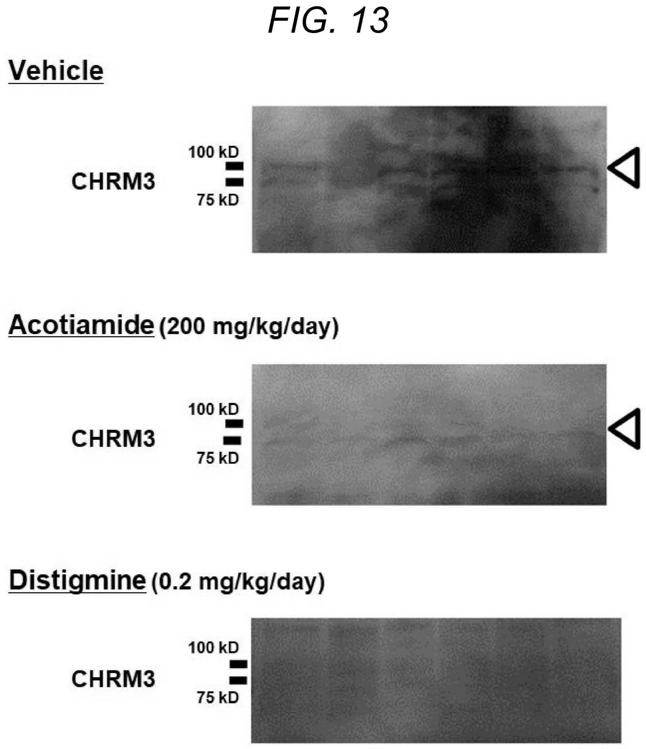

Fig. 13 shows results of the bladder M3 receptor expression level (protein) in Example **6.**

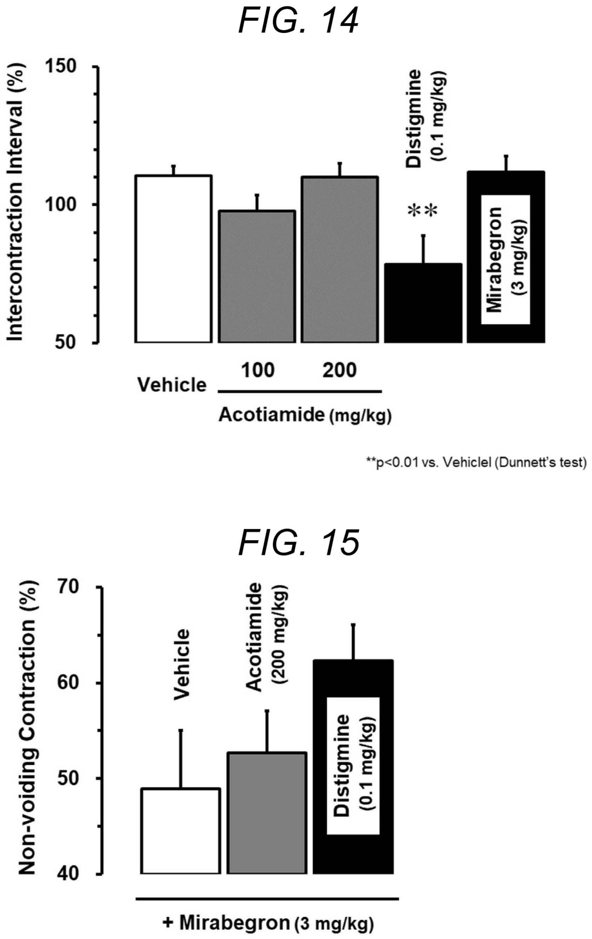

Fig. 14 shows results of a voiding interval in Example 7. In the drawing, the bar graph shows values of a vehicle administration group, acotiamide administration groups (100 and 200 mg/kg), a distigmine administration group (0.1 mg/kg), and a mirabegron administration group (3 mg/kg), respectively, from the left. The vertical axis represents the rate of change in voiding interval (intercontraction interval, %) (mean value and standard error of 6 to 7 animals in each group).
Fig. 15 shows results of a frequency of NVC of Example 7. In the drawing, the bar graph shows values of a vehicle administration group, an acotiamide administration group (200 mg/kg), and a distigmine administration group (0.1 mg/kg), respectively, from the left (under coadministration with mirabegron at 3 mg/kg). The vertical axis represents the rate of change in NVC (non-voiding contraction, %) (mean value and standard error of 7 animals in each group).

### Description of Embodiments

The present invention provides a urination disorder-alleviating agent comprising acotiamide or a salt thereof as an active ingredient.

According to a preferable aspect of the urination disorder-alleviating agent of the present invention, acotiamide or a salt thereof is orally administered in an amount of 200 to 300 mg per administration for a total of 600 to 900 mg per day to an adult.

As described above, the chemical name of acotiamide is N-[2-[bis(1-methylethyl)amino]ethyl]-2-[(2-hydroxy-4,5-dimethoxybenzoyl)amino]thiazole-4-carboxamide. Acotiamide is known to improve gastrointestinal motility disorders by significantly enhancing gastrointestinal motility (Patent Literature 1), and its hydrochloride hydrate (Acofide) is commercially available as a therapeutic agent for functional dyspepsia.

More specifically, the functional dyspepsia therapeutic agent containing acotiamide exhibits the efficacy and effect for postprandial fullness, upper abdominal bloating, and early satiation due to functional dyspepsia, and the administration and dosage thereof are to orally administer 100 mg of acotiamide hydrochloride hydrate per administration, three times a day, before meals, to an adult.

Examples of the active ingredient of the urination disorder-alleviating agent of the present invention include acotiamide and a salt thereof. Examples of the salt of acotiamide include acid addition salts, and specific examples thereof include acid addition salts with inorganic acids such as hydrochloride, sulfate, nitrate, phosphate, hydrobromide, and hydroiodide, and acid addition salts with organic acids such as acetate, oxalate, malonate, succinate, maleate, fumarate, lactate, malate, citrate, tartrate, methanesulfonate, and ethanesulfonate. Among them, acotiamide hydrochloride is more preferable. In addition, various solvates such as a hydrate of acotiamide or a salt thereof are also included. Acotiamide hydrochloride hydrate is further preferable. These acotiamide or salts thereof can be produced, for example, by the method described in Patent Literature **1.**

Regarding acotiamide or a salt thereof, the detrusor muscle contraction pressure, the urine flow rate, and the urethral pressure were examined by administration of a high dose greatly exceeding the dose exhibiting a gastrointestinal motility improving action, and it was found that acotiamide or a salt thereof does not affect the urethral pressure at a dose exhibiting a detrusor muscle contraction action, unlike the existing cholinergic agent.

As a result of verifying the effect of acotiamide or a salt thereof on the M3 receptor expression level in the bladder smooth muscle, it was found that the existing cholinergic agent decreases the M3 receptor expression level in the bladder by repeated administration, whereas acotiamide or a salt thereof does not decrease the M3 receptor expression level in the bladder by repeated administration. It was not previously reported and is unexpected that repeated administration of acotiamide or a salt thereof does not reduce the M3 receptor expression level in the bladder.

As a result of verifying the effect of acotiamide or a salt thereof on the voiding interval and the frequency of non-voiding contractions (non-voiding contraction, NVC) in the filling phase, it was found that in partial bladder outlet obstruction (BOO) rats exhibiting overactive bladder, the existing cholinergic agent shortens the voiding interval and increases the frequency of NVC in the filling phase, whereas acotiamide or a salt thereof does not affect the voiding interval and the frequency of NVC. It was not previously reported and is unexpected that acotiamide or a salt thereof does not affect the voiding interval or the frequency of NVC in an overactive bladder model.

Furthermore, it was found that the existing cholinergic agent reduces bladder compliance and induces cholinergic crisis at a dose close to the dose at which pharmacological effects occur, whereas acotiamide or a salt thereof does not affect bladder compliance and does not induce cholinergic crisis at a dose close to the dose at which pharmacological effects occur.

Here, these doses of acotiamide or a salt thereof correspond to an amount of 200 to 300 mg per administration for a total of 600 to 900 mg per day for oral administration for an adult.

Therefore, the urination disorder-alleviating agent comprising acotiamide or a salt thereof as an active ingredient of the present invention is preferably used such that acotiamide or a salt thereof is orally administered in an amount of 200 to 300 mg per administration for a total of 600 to 900 mg per day to an adult.

The number of oral administrations of acotiamide or a salt thereof per day is more preferably 2 to 3.

As described in Non-Patent Literatures 1 to 3, a pilot study was performed on a patient with underactive bladder using acotiamide (100 mg orally 3 times a day for 2 weeks) in combination with an α1-receptor blocker, and as a result, a sufficient decrease in residual urine volume was not obtained. In addition, as described in Non-Patent Literature 4, the efficacy and tolerability by high dose administration greatly exceeding the dose exhibiting a gastrointestinal motility improving action were not examined. In consideration of these facts, the effect of the therapeutic agent for urination disorder of the present invention could not be expected at all.

Examples of urination disorder to be treated with the therapeutic agent for urination disorder of the present invention include the above-described underactive bladder and underactive bladder accompanied by overactive bladder. Therefore, any of urination disorder due to underactive bladder, urination disorder due to overactive bladder, and urination disorder due to underactive bladder and overactive bladder is included. Specific symptoms include frequent urination, nocturia, urinary incontinence (urgent urinary incontinence, stress urinary incontinence, and the like), difficulty in urination, residual urine, urinary retention, and the like.

Acotiamide or a salt thereof exhibits efficacy when used alone as described above, or when used in combination with an agent having a urethral relaxation action at a different time or simultaneously. Here, examples of the drug having a urethral relaxation action that can be used in combination include urapidil, tamsulosin, naftopidil, silodosin, terazosin, and prazosin.

In addition, the urination-improving agent of the present invention provides a non-invasive treatment that is safe and effective as compared with existing cholinergic agents, thereby preventing transition to invasive treatments such as intermittent self-catheterization and indwelling catheterization (when the muscarinic M3 receptor is increased by acotiamide, the improvement of urinary function without relying on drugs is also expected). Furthermore, the urination-improving agent of the present invention enables treatment of urination disorder without worsening overactive bladder symptoms in patients with underactive bladder accompanied by overactive bladder (refer to Figs. 1 and 2).

The urination disorder-alleviating agent of the present invention is preferably a composition for oral administration obtained by blending a pharmaceutically acceptable carrier with acotiamide or a salt thereof. As the composition for oral administration, acotiamide or a salt thereof can be formulated into tablets, powders, granules, or capsules by appropriately using suitable additives such as excipients (for example, lactose, mannitol, corn starch, and crystalline cellulose), binders (for example, cellulose derivatives, gum arabic, and gelatin), disintegrants (for example, calcium carboxymethylcellulose), and lubricants (for example, talc and magnesium stearate). In addition, these solid preparations can be made into an enteric preparation using a coating base such as hydroxypropyl methylcellulose phthalate, hydroxypropyl methylcellulose acetate succinate, cellulose acetate phthalate, or a methacrylate copolymer.

### Examples

Next, the present invention will be described in more detail with reference to Examples, but the present invention is not limited to these Examples.

### Example 1 (Test of Bladder Using Normal Rats)

### Cystometry (detrusor muscle contraction pressure) and measurement of urine flow rate

### (1) Cystometry (detrusor muscle contraction pressure measurement)

A polyethylene catheter (PE50) for cystometry was inserted into and indwellingly placed in the bladder of male rats (strain: Crl:CD (SD), 8 weeks old, Jackson Laboratory Japan, Inc.). The catheter indwelling in the bladder was connected to a pressure transducer (Nihon Kohden Corporation) to measure the intravesical pressure. Physiological saline was injected into the bladder using an infusion pump, and changes in intravesical pressure were recorded on a polygraph system (RMT-1000, Nihon Kohden Corporation) and an analysis system (Lab Chart Pro, ADInstruments). Changes in detrusor muscle contraction pressure when distigmine (0.01 to 0.3 mg/kg), acotiamide (3 to 200 mg/kg), or vehicle was subcutaneously administered (5 mL/kg) were measured for 90 minutes.

### (2) Uroflowmetry (urine flow rate measurement)

Changes in urine flow rate when distigmine (0.1 mg/kg), acotiamide (30, 100, and 200 mg/kg) or vehicle was subcutaneously administered (5 mL/kg) to male rats (strain: Crl:CD (SD), 8 weeks old, Jackson Laboratory Japan, Inc.) were measured for 12 hours. Changes in urine flow rate were recorded using PowerLab (ADInstruments) by placing a digital balance under the voiding measurement cage to cumulatively collect excreted urine in a container on the balance and measure the change in weight.

### (3) Results

As shown in Figs. 3, 4, and 5, acotiamide increased the bladder contraction pressure during voiding and the maximum contraction pressure during voiding, and also showed an action of increasing the urine flow rate. The action of acotiamide on bladder contraction pressure was extremely strong in the 200 mg/kg administration group as compared with the 30 and 100 mg/kg administration groups. Meanwhile, distigmine increased the bladder contraction pressure during voiding and the maximum contraction pressure during voiding, but the urine flow rate decreased. Furthermore, distigmine significantly reduced bladder compliance, whereas acotiamide did not affect bladder compliance even at a dose that greatly increased detrusor muscle contraction pressure (200 mg/kg) (Fig. 6). Regarding the tolerability of the drug in the cystometry test, acotiamide was well tolerated up to the highest dose (200 mg/kg), but the highest dose of distigmine (0.3 mg/kg) induced cholinergic crisis (hypersecretion, respiratory abnormalities, convulsions (clonic/tonic convulsions)), and the tolerability was extremely low.

From the above results, it is considered that distigmine increases the detrusor muscle contraction pressure, but decreases the bladder compliance and does not exhibit an action of increasing the urine flow rate. Cholinergic crisis was induced at a dose close to the dose at which pharmacological effects occur, and the tolerability was extremely low. Meanwhile, acotiamide increases the detrusor muscle contraction pressure, and showed a strong action at the highest dose (200 mg/kg), but unlike distigmine, acotiamide does not affect the bladder compliance, and is considered to exhibit an action of increasing the urine flow rate. Furthermore, acotiamide did not induce cholinergic crisis even when the highest dose (200 mg/kg) was administered, and unlike distigmine, also exhibited good tolerability.

### Example 2 (Test for Confirming Tolerability)

### Investigation on induction of cholinergic crisis (rat)

Distigmine (0.1, 0.2, 0.3, and 0.4 mg/kg) or acotiamide (200 and 300 mg/kg) was subcutaneously administered to female rats (strain: Crl: CD (SD), Jackson Laboratory Japan, Inc.) (5 mL/kg) under urethane anesthesia, and the presence or absence of induction of cholinergic crisis was observed up to 120 minutes after administration.

Cholinergic crisis (hypersecretion, respiratory abnormalities) was induced following administration of distigmine, with 1 out of 3 animals in the 0.2 mg/kg group, 5 out of 6 animals in the 0.3 mg/kg group, and all animals (3 out of 3) in the 0.4 mg/kg group resulting in death.

Acotiamide did not induce cholinergic crisis at any dose, and no death cases were observed.

From the above results, distigmine induced cholinergic crisis at doses close to the dose at which pharmacological effects occur, and its tolerability was extremely low. Meanwhile, since acotiamide exhibited good tolerability even when high doses including the dose (200 mg/kg) that exerts strong pharmacological action were administered, the potential of acotiamide to induce cholinergic crisis was considered to be low.

### Example 3 (Test of Urethra Using Normal Rats)

### Measurement of LPP (Urethral Pressure)

Using female rats (strain: Crl:CD (SD), 9 weeks old, Jackson Laboratory Japan, Inc.), under isoflurane anesthesia, the spinal cord was transected at the Th8 vertebra, and after ligating and excising the ureters, a catheter (Becton Dickinson: PE-90, PE-50) was inserted and fixed at the bladder dome to prepare the measurement animal. Physiological saline was injected from the catheter, and the maximum intravesical pressure (leak point pressure (LPP)) when leaking from the external urethral opening was recorded by PowerLab (ADInstruments). Changes in leak point pressure when distigmine (0.1 mg/kg), bethanechol (3 mg/kg), acotiamide (30 to 300 mg/kg), or a vehicle was subcutaneously administered (5 mL/kg) were measured.

Acotiamide and distigmine did not change the leak point pressure and did not affect the urethral pressure (Fig. 7). Meanwhile, bethanechol showed an increase in leak point pressure and increased urethral pressure.

### Example 4 (Test of Bladder Using Normal Guinea Pigs)

### Cystometry (detrusor muscle contraction pressure) and measurement of urine flow rate

A polyethylene catheter (Becton Dickinson: PE50) was inserted into and fixed to the bladder of a guinea pig (male, strain: Slc:Hartley, 7 weeks old, Japan SLC, Inc.). A catheter indwellingly placed in the bladder was connected to a pressure transducer to measure intravesical pressure. Physiological saline was injected into the bladder using an infusion pump and intravesical pressure was recorded in PowerLab (ADInstruments). The urine flow rate was recorded in PowerLab (ADInstruments) by placing a digital balance under the cage to cumulatively collect excreted urine in a container on the balance and measure the change in weight. Changes in the detrusor muscle contraction pressure and the urine flow rate when distigmine (1 mg/kg), acotiamide (0.1 to 10 mg/kg), or a vehicle was intravenously administered (1 mL/kg) were measured for 30 minutes.

Distigmine increased the bladder contraction pressure during voiding and the maximum contraction pressure during voiding, but decreased the urine flow rate (maximum urine flow rate and average urine flow rate). Acotiamide increased the bladder contraction pressure during voiding and the maximum contraction pressure during voiding, and also showed an action of increasing the urine flow rate at the same dose (detrusor muscle contraction pressure; Fig. **8****,** Fig. **9****,** urine flow rate; Fig. 10, Fig. 11).

From the above results, it is considered that distigmine increases the detrusor muscle contraction pressure, but exhibits an action of decreasing the urine flow rate. Meanwhile, acotiamide increases the detrusor muscle contraction pressure, but unlike distigmine, it is considered that acotiamide exhibits an action of increasing the urine flow rate.

### Example 5 (Test of Bladder Using Urination Disorder [Underactive Bladder] Model Rats)

### Measurement of cystometry (detrusor muscle contraction pressure)

Under isoflurane anesthesia, female rats (strain: Crl:CD (SD), 8 weeks old, Jackson Laboratory Japan, Inc.) were subjected to pelvic nerve injury surgery to prepare pelvic nerve crush (PNC) rats. Cystometry was performed while awake, 2 to 4 weeks after surgery. A polyethylene catheter (PE50) for cystometry was inserted into the bladder and indwellingly placed. The catheter indwelling in the bladder was connected to a pressure transducer (Nihon Kohden Corporation) to measure the intravesical pressure. Physiological saline was injected into the bladder using an infusion pump, and changes in intravesical pressure were recorded in a polygraph system (RMT-1000, Nihon Kohden Corporation) (RMT-1000MG, Miyuki Giken Co., Ltd.) and an analysis system (Lab Chart Pro, ADInstruments).

Changes in detrusor muscle contraction pressure when distigmine (0.01 to 0.3 mg/kg), acotiamide (3 to 200 mg/kg), or vehicle was subcutaneously administered (5 mL/kg) to PNC rats were measured for 60 minutes.

Acotiamide and distigmine increased the detrusor muscle contraction pressure (Fig. 12). The action of acotiamide on the detrusor muscle contraction pressure was extremely strong in the 200 mg/kg administration group as compared with the 30 and 100 mg/kg administration groups.

Regarding drug tolerability, acotiamide showed good tolerability up to the highest dose, whereas at the highest dose of distigmine (0.3 mg/kg), cholinergic crisis (hypersecretion, respiratory abnormality, convulsions [clonic/tonic convulsions]) was induced.

From the above results, in the underactive bladder model, distigmine increased the detrusor muscle contraction pressure, but induced cholinergic crisis at doses close to the dose at which pharmacological effects occur. Meanwhile, acotiamide increased the detrusor muscle contraction pressure, but unlike distigmine, did not cause cholinergic crisis.

### Example 6 (Receptor Expression (Normal Rats))

### (Gene and Protein Expression Analysis)

Distigmine (0.1 mg/kg), acotiamide (100 mg/kg), or vehicle was subcutaneously administered twice daily for two weeks (5 mL/kg per dose) to normal rats (male, strain: Crl:CD (SD), 8 weeks old, Jackson Laboratory Japan, Inc.), and the bladder was collected. The M3 receptor expression level in the collected bladder was examined by gene quantitative analysis by real-time PCR (Chrm3) and protein expression analysis by Western blotting (CHRM3).

The results of the bladder M3 receptor expression level (gene) are shown in Table **1.** The results of the bladder M3 receptor expression level (protein) are shown in Fig. 13.

Repeated administration of distigmine reduced the expression level of the bladder M3 receptor. Meanwhile, the repeated administration of acotiamide did not reduce the expression level of the bladder M3 receptor.

**[Table 1]**

| **Group** | **Dose (mg/kg/day)** | **Number of Animals** | **Relative gene expression of Chrm3 (Mean ± SE)** |
|---|---|---|---|
| **Vehicle** | **-** | **6** | **106.3 ± 15.5** |
| **Acotiamide** | **200** | **6** | **107.0 ± 12.9** |
| **Distigmine** | **0.2** | **6** | **79.1 ± 11.8** |

### Example 7 (Test of Bladder Using Partial BOO Model Rats)

Voiding interval and frequency of non-voiding contractions (non-voiding contraction, NVC) in the filling phase

Under isoflurane anesthesia, female rats (strain: Crl:CD (SD), 6 weeks old, Jackson Laboratory Japan, Inc.) were subjected to bladder outlet obstruction surgery to prepare partial bladder outlet obstruction (BOO) rats. The sham-operated group was operated similarly except for the partial BOO. Six weeks after surgery, cystometry was performed while awake. A polyethylene catheter (Becton Dickinson: PE50) for cystometry was inserted into and indwellingly placed in the bladder. The catheter indwelling in the bladder was connected to a pressure transducer (Nihon Kohden Corporation) to measure the intravesical pressure. Physiological saline was injected into the bladder using a syringe pump and changes in intravesical pressure were recorded in PowerLab (ADInstruments).

### (Therapeutic Effect Evaluation Using Partial BOO Rats)

### (1) Effect of Single Agent on Voiding Interval

Distigmine (0.1 mg/kg), acotiamide (100 and 200 mg/kg) or vehicle was subcutaneously administered (5 mL/kg), or a β3-adrenergic receptor agonist (β3 agonist mirabegron, a therapeutic agent for overactive bladder) (3 mg/kg) was intravenously administered (1 mL/kg), and the voiding interval was measured for up to 2 hours.

### (2) Combination with β3 Agonist

The β3 agonist mirabegron (3 mg/kg, intravenous administration [1 mL/kg]) was co-administered with distigmine (0.1 mg/kg) or acotiamide (200 mg/kg) by subcutaneous administration (5 mL/kg), and the frequency of NVC was measured.

In the partial BOO rats (6 weeks after surgery), the frequency of NVC in the filling phase was increased compared to the sham-operated rats, and overactive bladder was exhibited.

For the effect of a single agent in the partial BOO rats, distigmine significantly shortened the voiding interval, while acotiamide and the β3 agonist had no effect on the voiding interval (Fig. 14).

Regarding the combined effect with the β3 agonist in the partial BOO rats, the NVC frequency increased in the distigmine combined use group as compared with the β3 agonist single administration group. The NVC frequency of the acotiamide combined use group was different from that of the distigmine combined use group, and was similar to that of the β3 agonist single administration group (Fig. 15).

From the above results, it was shown that in the partial BOO rats exhibiting overactive bladder, distigmine shortens the voiding interval and increases the frequency of NVC in the filling phase even under combined use of the β3 agonist, whereas acotiamide does not affect the voiding interval and the frequency of NVC.

### Example 8 (Clinical Trial)

Acotiamide at 600 to 900 mg per day or placebo is administered for 12 weeks to male or female patients with symptoms of underactive bladder.

As long as the dosage and administration are not changed during the clinical trial, an α1-blocker, a therapeutic agent for benign prostatic hyperplasia, a diuretic, an agent having an anticholinergic effect, and a Chinese medicine to be administered for the purpose of improving urinary symptoms, may be administered simultaneously with or at different times from acotiamide.

During clinical trials, as items related to the efficacy of the present agent, the International Prostate Symptom Score commonly used to measure symptoms of patients with underactive bladder, uroflowmetry parameters (voided volume, voiding time, maximum urine flow rate, average urine flow rate), post-void residual urine volume, voiding efficiency, parameters recorded in the bladder diary (urine discharge time, urine volume, presence or absence of a sense of urgency, presence or absence of slow stream, presence or absence of retarded micturition, and presence or absence of straining to void) are investigated.

During the execution of the clinical trial, as items related to the safety of the present agent, clinical examinations (hematology, blood biochemistry, urine general tests), vital signs, 12 lead electrocardiography, and the occurrence of adverse events are investigated.

## Claims

1. A urination disorder-alleviating agent comprising acotiamide or a salt thereof as an active ingredient, wherein the acotiamide or a salt thereof is orally administered in an amount of 200 to 300 mg per administration for a total of 600 to 900 mg per day to an adult.

2. The urination disorder-alleviating agent according to claim **1,** wherein an administration subject is a patient selected from the group consisting of patients with underactive bladder and patients with underactive bladder accompanied by overactive bladder.

3. The urination disorder-alleviating agent according to claim **1,** wherein the number of oral administrations of acotiamide or a salt thereof per day is 2 to **3.**

4. The urination disorder-alleviating agent according to claim **1,** wherein an administration subject is a patient with underactive bladder.

5. Use of acotiamide or a salt thereof for producing a urination disorder-alleviating agent, wherein the acotiamide or a salt thereof is orally administered in an amount of 200 to 300 mg per administration for a total of 600 to 900 mg per day to an adult.

6. The use according to claim 5, wherein an administration subject is a patient selected from the group consisting of patients with underactive bladder and patients with underactive bladder accompanied by overactive bladder.

7. The use according to claim 5, wherein the number of oral administrations of acotiamide or a salt thereof per day is 2 to 3.

8. The use according to claim 5, wherein an administration subject is a patient with underactive bladder.

9. Acotiamide or a salt thereof for use in alleviating urination disorder, wherein the acotiamide or a salt thereof is orally administered in an amount of 200 to 300 mg per administration for a total of 600 to 900 mg per day to an adult.

10. The acotiamide or a salt thereof according to claim **9,** wherein an administration subject is a patient selected from the group consisting of patients with underactive bladder and patients with underactive bladder accompanied by overactive bladder.

11. The acotiamide or a salt thereof according to claim 9 or 10, wherein the number of oral administrations per day is 2 to 3.

12. The acotiamide or a salt thereof according to any one of claims 9 to 11, wherein an administration subject is a patient with underactive bladder.

13. A method for alleviating urination disorder, comprising orally administering acotiamide or a salt thereof in an amount of 200 to 300 mg per administration for a total of 600 to 900 mg per day to an adult.

14. The method according to claim 13, wherein an administration subject is a patient selected from the group consisting of patients with underactive bladder and patients with underactive bladder accompanied by overactive bladder.

15. The method according to claim 13, wherein the number of oral administrations of acotiamide or a salt thereof per day is 2 to 3.

16. The method according to claim 13, wherein an administration subject is a patient with underactive bladder.
